Europäisches Patentamt

European Patent Office

Office européen des brevets

(19)

(11) Veröffentlichungsnummer: **0 217 740**
**B1**

(12) **EUROPÄISCHE PATENTSCHRIFT**

(45) Veröffentlichungstag der Patentschrift:
07.03.90

(51) Int. Cl.⁴: **B25C 11/02**

(21) Anmeldenummer: 86810331.8

(22) Anmeldetag: 24.07.86

(54) Werkzeug zum Herausziehen von Heftlammern.

(30) Priorität: 30.08.85 CH 3755/85

(43) Veröffentlichungstag der Anmeldung:
08.04.87 Patentblatt 87/15

(45) Bekanntmachung des Hinweises auf die Patenterteilung:
07.03.90 Patentblatt 90/10

(84) Benannte Vertragsstaaten:
AT BE CH DE FR GB IT LI NL SE

(56) Entgegenhaltungen:
DE-B- 1 077 634
US-A- 499 637
US-A- 2 662 727
US-A- 4 036 471

(73) Patentinhaber: Schneider-Muro, Karl Walter, in der Breiti 13, CH-8047 Zürich(CH)

(72) Erfinder: Schneider-Muro, Karl Walter, in der Breiti 13, CH-8047 Zürich(CH)

(74) Vertreter: Feldmann, Paul David et al, c/o Patentanwaltsbüro FELDMANN AG Kanalstrasse 17, CH-8152 Glattbrugg(CH)

**Beschreibung**

Die Erfindung bezieht sich auf ein Werkzeug zum Herausziehen von Heftklammern, die in eine feste Unterlage eingeschlagen sind. Bisher hat man dazu eine Ahle, einen Pfriem oder einen Schraubenzieher verwendet, den man unter die Heftklammer drückte oder schlug um sie anschliessend mit demselben Werkzeug heraus zu hebeln. Bei dieser Hebelwirkung wird die Unterlage oft verletzt und bekommt hässliche Druckstellen, die besonders störend sind, wenn dieselbe Unterlage wieder verwendet wird.

Dies kommt aber regelmässig bei Polstermöbeln vor, wenn sie neu Ueberzogen werden Dabei handelt es sich zudem um eine grosse Anzahl von Heftklammern die zuerst sorgfältig entfernt werden müssen, bevor der neue Ueberzug angebracht werden kann.

Bei Polstermöbeln sind nämlich die Heftklammern parallel zur Zargenlängsrichtung eingeschlagen oder eingedrückt. Beim Heraushebeln mit Hilfe eines Pfriems oder Schraubenziehers wird dieses Behelfswerkzeug senkrecht zur Zargenlängsrichtung angesetzt, und lässt beim einfachen Heraushebeln hässliche Druckspuren im Rand der Zarge zurück.

Es sind Zangen zum Herausziehen oder Heraushebeln bekannt, bei denen die Zangenwirkung lediglich dazu dient, die herausgezogene Heftklammer zu halten, damit sie nicht auf den Boden fällt. So zeigt beispielsweise die EU-PS 059 778 ein Werkzeug bei dem ein Schenkel unter die eingeschlagene Heftklammer geschoben werden muss, wobei der andere Schenkel die herausgezogene Klammer hält. Die EU-PS 0l22 863 bezieht sich auf ein ähnliches Werkzeug, bei dem eine Hebelübersetzung dafür sorgt, dass die Klemmwirkung umso grösser ist, je grösser die Kraft zum Heraushebeln ist.

Für den Bürogebrauch sind einfache Werkzeuge zum Herausziehen von Heftklammern bekannt. Ein derartiges Werkzeug hat zwei Paare von in geschlossenen Zustand dicht nebeneinander liegenden, sich überschneidenden spitzen Greifzähnen. Es sind hier zwei Paare erforderlich, weil diese die umgebogenen Schenkel der Heftklammer wieder senkrecht zum Steg zurückbiegen müssen.

Die US-PS 499 637 (Knight) zeigt eine Zange zum Herausziehen einer Heftklammer aus einer festen Unterlage. Ihr Maul ist mit einem spitzen Zahn versehen, hinter dem eine halbrunde Ausnehmung ist. Beim Schliessen des Maules greift ein zweiter Zahn in diese Ausnehmung ein. In geschlossenem Zustand des Maules verbleibt hinter den ineinandergreifenden Zähnen eine Öffnung frei. Dadurch besteht die Gefahr, daß nur ein Schenkel der Heftklammer aus der Unterlage herausgezogen wird. Eine ganz herausgezogene Heftklammer wird nicht von der Zange gehalten. Hinter der halbrunden Öffnung befindet sich an den Maulhälften eine Klemmfläche, die aber keineswegs eine herausgezogene Heftklammer automatisch zu greifen vermag.

Die oben genannten Werkzeuge weisen denselben Nachteil auf, wie eine behelfsmässig verwendeter Pfriem oder Schraubenzieher, indem die Hebelwirkung senkrecht zur Längsachse der Heftklammer erfolgt.

Die Erfindung hat sich die Aufgabe gestellt ein Werkzeug zu schaffen, dass diese Arbeit erleichtert, beschleunigt und zugleich die Unterlage schont.

Die Erfindung löst diese Aufgabe mit einem Werkzeug, das die Merkmale des Patentanspruches l aufweist. Nachdem die Greifzähne den Steg der Heftklammer gefasst haben, gleitet dieser entlang den Zähnen und wird von der an die Zähne anschliessenden Klemmfläche gefasst und festgehalten. Dadurch werden mit Sicherheit beide Schenkel herausgezogen.

Die Klemmwirkung der Klemmfläche erlaubt aber auch die Zange, nachdem sie den Steg der Klammer gefasst hat ein wenig zu drehen, so dass auch mit einer Zange, bei der der Kippwulst in der Schwenkebene der Zangengriffe verläuft die Heftklammern eines Polstermöbels unter Kippwirkung herausgezogen werden können, ohne den Rand der Zarge zu verletzen. Dies ist natürlich nur bei einer schmalen Zarge erforderlich.

Das erfindungsgemässe Werkzeug erlaubt, insbesondere bei Polstermöbeln, die vielen Heftklammern auf rationelle Weise aus der Zarge herauszuziehen, ohne dass auf dem Rand der Zarge Druckspuren zurückbleiben. Die Kippstütze ermöglicht nämlich ein Herausziehen der Heftklammern durch Kippwirkung, wobei sich die Stütze nicht am Rand der Zarge, sondern auf der Fläche der Zarge abstützt.

In der beigefügten Zeichnung sind einige Ausführungsbeispiele des Erfindungsgegenstandes dargestellt. Es zeigen:

Figur l ein Werkzeug in Seitenansicht;
Figur 2 das Werkzeug nach Figur l in Ansicht von der Schmalseite
Figur 3 das Werkzeug nach Figur l in geöffnetem Zustand
Figur 4 eine Vorderansicht der Greifzähne des Werkzeuges nach Figur l
Figur 5 das geschlossene Maul eines zweiten Werkzeuges
Figur 6 das geöffnete Maul des Werkzeuges nach Figur 5 von vorne
Figur 7 das geschlossene Maul des Werkzeuges nach Figur 5 von vorne
Figur 8 das Werkzeug nach Figur 5 beim Gebrauch
Figur 9 eine Variante zum Werkzeug nach Figur l
Figur 9a ein Detail des Werkzeuge nach Figur 9
Figur l0a-c erläutern den Gebrauch des Werkzeuges nach Figur 9 in drei Phasen

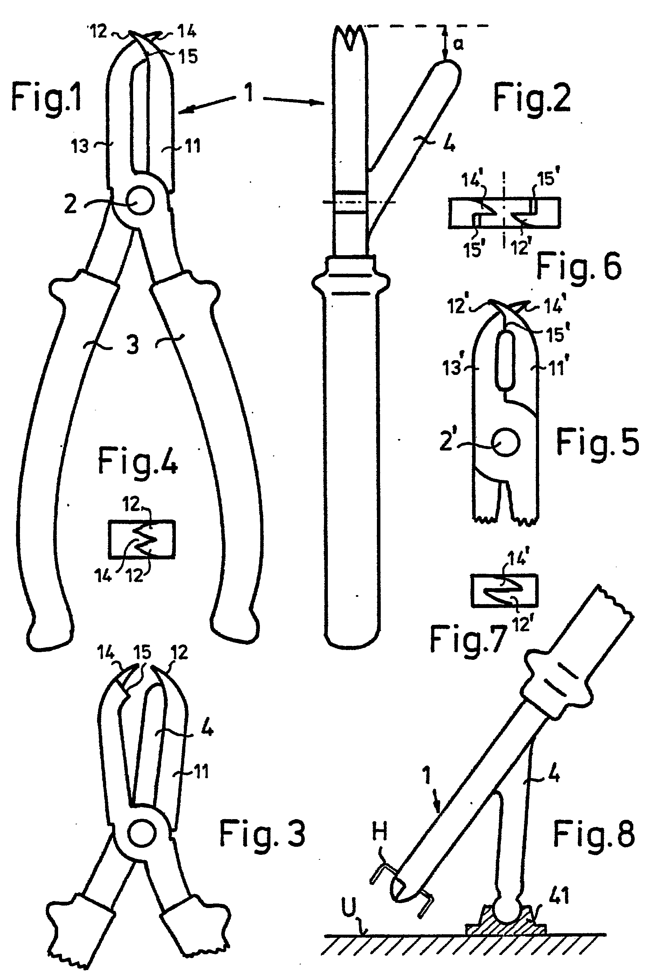

Das Werkzeug nach Figur l-4 hat die Form einer Zange mit einem Maul l, einem Gelenk 2 und zwei Griffen 3 .Es unterscheidet sich aber von bekannten Zangen dadurch, dass die rechte Maulhälfte ll, zwei spitze Greifzähne l2 hat, während die linke Maulhälfte l3 einen spitzen Greifzahn l4, hat der zwischen die Zähne l2 greift. In geschlossenem Zustand überschneiden sich die Zähne l2 und l4 wie dies in Figur l und Figur 4 ersichtlich ist. Ferner weist die Zange eine, im Bereich des Gelenks 2 angebrachte, schräg nach vorne gerichtete Kippstütze 4 auf. Die Kippstütze ist fest mit dem Maulteil I und dem linken Griff 3 verbunden. Sie steht um einen gewissen Abstand a gegen die Greifzähne zurück. An der linken Maulhälfte I3 ist eine an den Zahn I4 anschliessende Klemmfläche I5 angebracht, die in geschlossenem Zustand auf der Maulhälfte II aufliegt.

Beim Herausziehen einer Heftklammer aus der Unterlage gleitet sie an den Zähnen entlang und wird dann zwischen den Flächen I5 der Maulhälfte I3 und der Maulhälfte II klemmend gehalten wie dies in Figur 8 dargestellt ist.

Figur 3 zeigt die Zange mit geöffnetem Maul. Daraus ist ersichtlich, dass die Kippstütze 4 mit der rechten Maulhälfte mitschwenkt. Da aber die Heftklammern nur eine geringe Dicke aufweisen, ist das Maul der Zange wieder annähernd geschlossen sowie die Zähne die Heftklammer gepackt haben und die Klammer zwischen den Flächen I5 geklemmt gehalten ist.

Die Figuren 5-7 zeigen das Maul einer anderen Zange, die nur zwei spitze Greifzähne I2',I4' aufweist. Das starke Ende dieser Greifzähne geht in die Auflagefläche I5' der Maulteile II',I3' über.

Figur 8 zeigt diese Zange im Betrieb, beim Herausziehen einer Heftklammer H aus der Unterlage U. Dabei haben die spitzen Greifzähne zuerst die Heftklammern untergriffen. Die Klammer gleitet, sowie sie aus der Unterlage herausgezogen ist, an den spitzen Zähnen entlang nach rückwärts und wird zwischen den Klemmflächen I5 festgehalten. Zum Herausziehen der Klammer wurde die Zange um die Kippstütze 4 gekippt, was diese Arbeit sehr erleichtert.

Bei diesem Werkzeug ist die Kippstütze 4 an ihrem freien Ende mit einem Fuss 4I versehen, der die Auflagefläche beim Herausziehen der Heftklammern vergrössert. Dadurch wird der Druck über eine grössere Fläche verteilt und die Gefahr einer Verletzung einer relativ weichen Unterlage verringert. Der Fuss ist gelenkig mit der Stütze verbunden, wozu das freie Ende derselben kugelförmig gearbeitet ist. Bei entsprechender Ausgestaltung kann der Fuss auch als Verlängerung der Stütze dienen. Für Heftklammern mit relativ langen Schenkeln, wie sie in weichen Unterlagen verwendet werden, ist dies ein wesentlicher Punkt. Der Fuss 4I kann aus Metall oder als aufsteckbaren Aufsatz aus Kunststoff gefertigt sein.

Statt die Kippstütze im Bereich des Gelenkes anzubringen, kann sie auch an einer oder an beiden Maulhälften angebracht sein. Je kleiner der Abstand zwischen der Stütze und den spitzen Greifzähnen ist, umso kleiner ist der Hebelarm und umso geringer ist die zum Heraushebeln benötigte Kraft. Allerdings verringert sich dadurch auch die Möglichkeit lange Heftklammern noch mit Sicherheit durch alleinige Hebelwirkung ganz herausziehen zu können.

Aus ergonometrischen Gründen hat sich gezeigt, dass es von Vorteil ist, wenn die beiden Schenkel 3 der Zange möglichst nahe beim Gelenk 2 in Richtung zur Kippstütze abgekröpft sind. Der Benützer muss folglich den Arm um den Abkröpfungswinkel weniger verdrehen. Zudem hat er eine bessere Hebelwirkung, weil die senkrechte Distanz zwischen Angriffspunkt der Zange weiter vom Auflagepunkt der Kippstütze entfernt ist, beim Beginn der Kippbewegung.

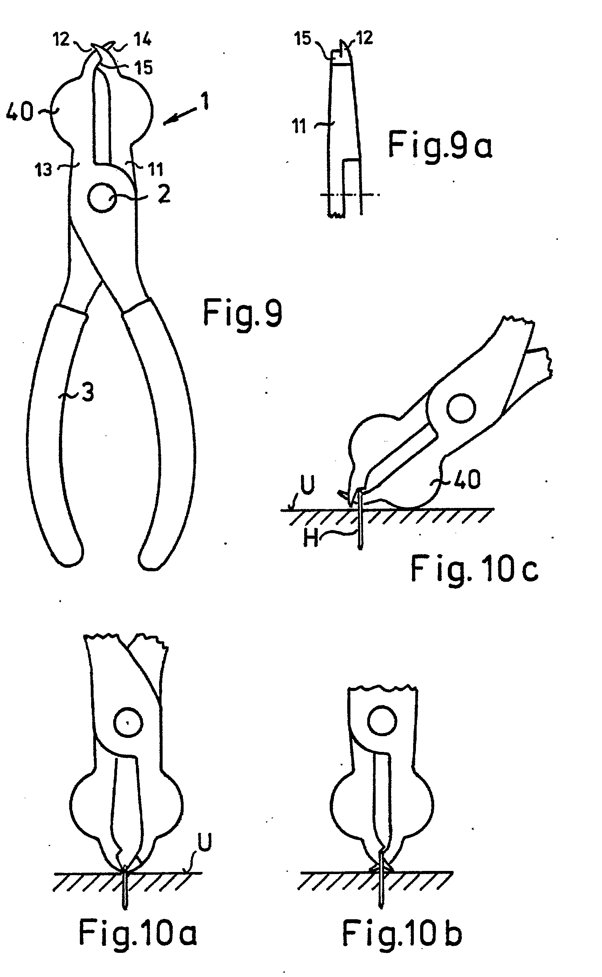

Figur 9 zeigt eine Zange die sich vor der vorgehend beschriebenen Zange nach Figur I dadurch unterscheidet, dass das Kippstützelement 40 auf der Aussenseite der Maulteile II,I3 angebracht ist und in der Schwenkebene der Griffe 3 verläuft. Figur 9a zeigt einen Maulteil II in Ansicht von innen her. Hier ist die Klemmfläche I5 deutlich erkennbar.

Diese Zange kann mit ihren Greifzähnen I2,I4 eine Heftklammer H herausziehen wie vorgehend beschrieben. Die Die Wirkungsweise geht in drei Phasen vor sich:

a) Zange öffnen, auf die Unterlage drücken und zudrücken, wobei die Greifzähne I2,I4 unter die Heftklemmer H greifen.
(Figur I0a)

b) die Zange weiter zudrücken, wobei der Steg der Heftklammer entlang der Zähnen I2, I4 nach oben gleitet und schliesslich zwischen den beiden Klemmflächen festgeklemmt wird. Es spielt dabei keine Rolle ob nur ein Schenkel aus dem Untergrund U herausgezogen wurde und der zweite Schenkel noch fessteckt. (Figur I0b)

c) Jetzt wird die Zange in geschlossenem Zustand um das Kippstützelement 40 gekippt wie dies Figur I0c zeigt und die Heftklammer weiter herausgezogen, unabhängig davon, ob noch ein Schenkel im Untergrund steckt oder nicht.

Beim Herausziehen von Heftklammern aus einer schmalen Zarge eines Polstermöbels muss unter Umständen zuerst die Zarge in der Lage nach Figur I0b ein wenig gedreht werden, damit das Kippstützelement beim nachfolgenden Heraushebeln den Rand der Zarge nicht verletzt.

**Patentansprüche**

1. Werkzeug in Form einer Zange zum Herausziehen von Heftklammern, die in eine feste Unterlage eingeschlagen sind mit einem Maul (1), das vorne mindestens zwei, in geschlossenem Zustand dicht nebeneinander liegende sich überschneidende, spitze Greifzähne aufweist und mit mindestens einem, im Bereich des Maules angeordneten, gegen die Greifzähne (12, 14) zurückstehenden Kippstützelement (4, 40), dadurch gekennzeichnet, daß die spitzen sich überschneidenden Greifzähne (12, 14) so angeordnet und ausgebildet sind, daß deren Flanken sich beim Schließen des Maules aufeinander gleitend bewegen und daß anschließend an das dicke Ende der spitzen Zähne, an der Maulinnenseite Klemmflächen (15) angeordnet sind, die bei geschlossenem Maul aufeinanderliegen, so daß diese Klemmflächen den Steg einer von den spitzen Zähnen erfaßten, mindestens teilweise herausgezogenen Heftklammer zu halten vermögen.

2. Werkzeug nach Anspruch I, dadurch gekennzeichnet, dass das Kippstützelement an einem Maul-

teil (II) im Bereich des Gelenkes (2) der Zange angebracht ist, schräg nach vorne ragt und in einer Ebene senkrecht zur Schwenkebene der Zangengriffe (3) verläuft.

3. Werkzeug nach Anspruch I, dadurch gekennzeichnet, dass das Kippstützelement in Form einer Wulst (40) auf der Aussenseite mindestens eines Maulteiles (II,I3) angebracht ist und in der Schwenkebene der Zangengriffe (3) verläuft.

4. Werkzeug nach Anspruch I, dadurch gekennzeichnet, dass ein Backen (II) des Maules zwei Greifzähne (I2), der andere Backen nur einen einzigen Greifzahn (I4) aufweist, der in geschlossenem Zustand des Maules zwischen den beiden andern Zähnen liegt.

5. Werkzeug nach Anspruch I, dadurch gekennzeichnet, dass jeder Backen nur einen Zahn aufweist und dass diese in geschlossenem Maul sich überschneiden und nahe beieinander liegen.

6. Werkzeug nach Anspruch I, dadurch gekennzeichnet, dass die Kippstütze an einem der Maulhälften angebracht ist.

7. Werkzeug nach Anspruch 3, dadurch gekennzeichnet, dass an beiden Maulhälften je eine Kippstütze angebracht ist.

8. Werkzeug nach Anspruch 2, dadurch gekennzeichnet, dass das freie Ende der Kippstütze mit einem, eine vergrösserte Stützfläche bildenden Fuss (4I) schwenkbar verbunden ist.

9. Werkzeug nach Anspruch I, dadurch gekennzeichnet, dass die beiden Griffe (3) der Zange, relativ nahe dem Gelenk (2) in Richtung zur Kippstütze (4) abgekröpft sind.

I0. Verwendung des Werkzeugs nach Anspruch 2, beim Herausziehen einer Heftklammer aus der Zarge eines Polstermöbels, bei dem die Heftklammern parallel zur Längsachse der Zarge eingeschlagen sind, dadurch gekennzeichnet, dass man mit senkrecht zur Zargenfläche gehaltenem Werkzeug, den Verbindungssteg der Heftklammer greift und diese durch Kippen auf der Zarge um das Kippstützelement herauszieht, wobei die Kippstütze sich auf der Zargenfläche abstützt.

## Claims

1. Tool in the form of pliers for extracting staples from a solid base, with a pair of jaws (1) which have at their tips at least two sharp, gripping teeth overlapping and fitting close together in the closed position, and with at least one rocking support (4, 40) located in the area of the jaws but set back from the teeth (12, 14), characterised in that the sharp overlapping teeth (13, 14) are so arranged and formed that their flanks slide past each other as the jaws are closed, and in that clamping surfaces (15) are provided at the thick ends of the sharp teeth on the inside of the jaws, said clamping surfaces being in contact with each other when the jaws are closed, so that these clamping surfaces can grip the bridge of a staple that has been engaged and at least partly removed by the sharp teeth.

2. Tool according to Claim I, characterised in that the rocking support is located on one jaw (11) in the area of the joint (2) of the pliers, and projects obliquely and forwardly in a plane perpendicular to the pivoting plane of the handles (3) of the pliers.

3. Tool according to Claim 1, characterised in that the rocking support is mounted to the outside of at least one of the jaws (11, 13) in the form of a lobe (40) extending in the pivoting plane of the handles (3) of the pliers.

4. Tool according to Claim 1, characterised in that one jaw (11) has two gripping teeth (12) and the other jaw only one gripping tooth (14), which is located between the other two teeth in the closed position of the jaws.

5. Tool according to Claim 1, characterised in that each of the jaws has only one tooth and that these teeth overlap and fit close together in the closed position of the jaws.

6. Tool according to Claim 1, characterised in that the rocking support is mounted to one half of the jaws.

7. Tool according to Claim 3, characterised in that a rocking support is mounted to each half of the jaws.

8. Tool according to Claim 2, characterised in that the free end of the rocking support is pivotally linked to a foot (41) forming an enlarged support area.

9. Tool according to Claim 3, characterised in that the two handles (3) of the pliers are cranked towards the rocking support (4) at a location relatively close to the joint (2).

10. Application of the tool according to Claim 2 when extracting a staple from the frame of a piece of upholstered furniture, and when the staples are inserted parallel to the longitudinal axis of the frame, characterised in that the bridge of the staple is gripped with the tool held perpendicularly to the frame surface and is then extracted by pivoting the tool on the frame about the rocking support, the rocking support being supported on the frame surface.

## Revendications

1. Instrument revêtant la forme d'une pince, en vue de l'extraction d'agrafes enfoncées dans un support sous-jacent rigide, comprenant une gueule (1) présentant, à l'avant, au moins deux dents de préhension pointues, qui s'entrecroisent et sont intimement juxtaposées à l'état fermé, ainsi qu'au moins un élément (4, 40) de soutien basculant, disposé au voisinage de la gueule et en retrait par rapport aux dents de préhension (12, 14), caractérisé par le fait que les dents de préhension (12, 14), pointues et s'entrecroisant, sont agencées et réalisées de telle sorte que leurs flancs se meuvent par glissement l'un sur l'autre lors de la fermeture de la gueule, et que des surfaces de coincement (15), situées à la face interne de la gueule dans la continuité de l'extrémité épaisse des dents pointues, reposent l'une sur l'autre lorsque la gueule est fermée, si bien que ces surfaces de coincement sont en mesure de retenir la membrure d'une agrafe saisie par les dents pointues, et extraite au moins en partie.

2. Instrument selon la revendication 1, caractérisé par le fait que l'élément de soutien basculant est implanté sur une partie (11) de la gueule au voisinage de l'articulation (2) de la pince, dépasse à l'oblique vers l'avant et s'étend dans un plan perpendiculaire au plan de pivotement des poignées (3) de la pince.

3. Instrument selon la revendication 1, caractérisé par le fait que l'élément de soutien basculant est ménagé, sous la forme d'un bourrelet (40), sur la face externe d'au moins une partie (11, 13) de la gueule, et s'étend dans le plan de pivotement des poignées (3) de la pince.

4. Instrument selon la revendication 1, caractérisé par le fait qu'une mâchoire (11) de la gueule comporte deux dents de préhension (12), l'autre mâchoire présentant une seule et unique dent de préhension (14) qui se trouve entre les deux autres dents à l'état fermé de la gueule.

5. Instrument selon la revendication 1, caractérisé par le fait que chaque mâchoire présente seulement une dent ; et par le fait que ces dernières s'entrecroisent et sont intimement rapprochées dans la gueule fermée.

6. Instrument selon la revendication 1, caractérisé par le fait que le soutien basculant est ménagé sur l'une des demi-gueules.

7. Instrument selon la revendication 3, caractérisé par le fait qu'un soutien basculant respectif est ménagé sur les deux demi-gueules.

8. Instrument selon la revendication 2, caractérisé par le fait que l'extrémité libre du soutien basculant est reliée, avec faculté de pivotement, à un pied (41) formant une surface d'appui agrandie.

9. Instrument selon la revendication 2, caractérisé par le fait que les deux poignées (3) de la pince sont recourbées en direction du soutien basculant (4), relativement près de l'articulation (2).

10. Application de l'instrument selon la revendication 2, lors de l'extraction d'une agrafe hors du châssis d'un meuble à rembourrage dans lequel les agrafes sont enfoncées parallèlement à l'axe longitudinal du châssis, caractérisée par le fait que, l'instrument étant tenu perpendiculairement à la surface du châssis, l'on saisit la membrure de solidarisation de l'agrafe et l'on extrait cette dernière par basculement, sur le châssis, autour de l'élément de soutien basculant, ce soutien basculant prenant appui sur la surface du châssis.

Fig.1

Fig.2

12
14
15
1
13
11
2
3
Fig.4

12
14 12

a
4

14'
15'
15'
12'

Fig. 6

12'
14'
15'
13'
11'
2'

Fig.5

14'
12'

Fig.7

14 15 12
4
11
Fig. 3

1
4
H
U
41
Fig.8

Fig.9a

Fig.9

Fig. 10 c

Fig.10 a

Fig.10 b